# EUROPEAN PATENT SPECIFICATION

(11) **EP 0 772 461 B1**
(45) Date of publication and mention of the grant of the patent: **14.11.2001**
(21) Application number: 95926012.6
(22) Date of filing: 21.07.1995
(51) Int. Cl.: A61L 2/06, B65B 3/00

(54) **Method of sterilizing a prefilled syringe**
Verfahren zum Sterilisieren einer vorgefüllten Spritze
Procédé de stérilisation d'une seringue remplie

(30) Priority: 21.07.1994 JP 16850494; 17.07.1995 JP 20288595
(43) Date of publication of application: 14.05.1997
(73) Proprietor: Bracco International B.V., 1077 ZX Amsterdam (NL)
(72) Inventor: NOZU, Yukio, Hashima-gun Gifu-ken 501-61 (JP); IKEUCHI, Takayuki, Konan-shi Aichi-ken 483 (JP); IDA, Katsumi, Honjo-shi Saitama-ken 367 (JP); WATANABE, Sumio, Niwa-gun Aichi-ken 480-01 (JP)
(74) Representative: Turi, Michael, Dipl.-Phys.
(86) International application number: JP9501459
(87) International publication number: WO9603156

(56) References cited:
- EP-A- 0 328 504
- US-A- 4 628 969

## Description

### TECHNICAL FIELD

The present invention relates to a method of sterilizing a syringe prefilled with a liquid medical substance (medication to be injected).

### BACKGROUND ART

In case of using a medical liquid substance sealed in an ample or vial as a medication (referred to merely as an injection hereinafter) to be injected to a human body, it is required to first open the ample or vial and then, to introduce the injection into a syringe from the ample or vial. However, in order to quickly open the ample or vial and correctly introduce the injection into the syringe, not only certain skill and experiences are required, but also complexity of procedure might cause a critical delay in the timing of dosage to a patient. Accordingly, such complexity should be eliminated, especially in a case of emergency. Furthermore, it is not always ensured that extraneous matters like fine glass powder as generated at the time of opening the ample or vial, never drop in and mix with the injection in the ample or vial. In order to obviate such problems which might happen unexpectedly, there has been disclosed, in a laid-open patent publication No. Sho 49-28194, a prefilled syringe which contains the injection in its outer cylindrical body having a nozzle at its one end and an opening at its other, said nozzle and opening being sealed with a cap and a gasket, respectively.

Needless to say, however, this prefilled syringe has to be sterilized before practical use of it. In this case, sterilization is applied to the entirety of the prefilled syringe, in other words the syringe is sterilized in such a state that the cap and the gasket are fitted to the nozzle and the opening, respectively. In general, sterilization of this sort is carried out with steam as is highly elevated in its temperature and pressure by using an autoclave. In case, however, the nozzle part is complicatedly designed in shape, steam for sterilization fails in creeping into a tiny gap between the inner surface of the cap and the outer surface of the nozzle. Also in the case the gasket shape is complicated, sterilization steam can not easily creep into the gap between the circumferential surface of the gasket and the inner wall surface of the syringe. Consequently, in case the cap and the gasket are complicated in shape, it is hardly possible to adequately sterilize the prefilled syringe under such a condition (i.e. sterilization time and temperature) that is usually adopted in sterilization of the prefilled syringe by using the autoclave, so that there happens a case where the time for sterilization is obliged to be extended or the temperature for the same is obliged to be elevated excessively.

The document US 4,628,969 shows a sterilizing method according to the preamble of claim 1, in particular wetting of the surfaces of all parts of the syringe to be assembled in order to provide the formation of steam during the sterilization. However, a long sterilization time is needed in the subsequent final sterilization of the prefilled syringe due to the evaporation of the liquid film of the wetted surfaces.

Accordingly, an object of the present invention is to provide a method of sterilizing a prefilled syringe wherin adequate sterilization can be performed throughout the gap between the inner surface of the cap and the outer surface of the nozzle as well as the gap between the circumferential surface of the gasket and the inner wall of the syringe, in particular by optimizing the overall sterilization time.

### DISCLOSURE OF THE INVENTION

According to the present invention as recited in claim 1, there is provided a method of sterilizing a prefilled syringe having an outer cylindrical body which includes a nozzle which is integrally formed at its one end and an opening which is provided at its other end. Classified according to the assembly order of cap, gasket and body and to the specific wetting method, the inventive method comprises the following steps:
- sealing the nozzle of said outer cylindrical body by means of a cap, introducing an injection into the inside of said outer cylindrical body, fabricating the prefilled syringe by sealing the opening of said outer cylindrical body with a gasket, and sterilizing the entirety of said prefilled syringe as fabricated, wherein the surface of said cap is made to wet, before sealing the nozzle of said outer cylindrical body with said cap, by either way of immersing said cap in distilled water, or exposing said cap to steam, or applying steam sterilization to said cap as is being kept in distilled water, and the surface of said gasket is made to wet, before sealing the opening of said outer cylindrical body with said gasket, by either way of immersing said gasket in distilled water, or exposing said gasket to steam, or applying steam sterilization to said gasket as is being kept in distilled water;
- sealing the opening of said outer cylindrical body by means of a gasket, introducing an injection into the inside of said outer cylindrical body, fabricating the prefilled syringe by sealing the nozzle of said outer cylindrical body with a cap, and sterilizing the entirety of said prefilled syringe as fabricated, wherein the surface of said gasket is made to wet, before sealing the opening of said outer cylindrical body with said gasket, by either way of immersing said gasket in distilled water, or exposing said gasket to steam, or applying steam sterilization to said gasket as is being kept in distilled water, and the surface of said cap is made to wet, before sealing the nozzle of said outer cylindrical body with said cap, by either way of immersing said cap in distilled water, or exposing said cap to steam, or applying steam sterilization to said cap as is being kept in distilled water;
- sealing the nozzle of said outer cylindrical body by means of a cap, introducing an injection into the inside of said outer cylindrical body, fabricating the prefilled syringe by sealing the opening of said outer cylindrical body with a gasket, and sterilizing the entirety of said prefilled syringe as fabricated, wherein said cap is sterilized with steam after being immersed in distilled water, before sealing the nozzle of said outer cylindrical body therewith, and said gasket is sterilized with steam after being immersed in distilled water, before sealing the opening of said outer cylindrical body therewith; or
- sealing the opening of said outer cylindrical body by means of a gasket, introducing an injection into the inside of said outer cylindrical body, fabricating the prefilled syringe by sealing the nozzle of said outer cylindrical body with a cap, and sterilizing the entirety of said prefilled syringe as fabricated, wherein said gasket is sterilized with steam after being immersed in distilled water, before sealing the opening of said outer cylindrical body therewith, and said cap is sterilized with steam after being immersed in distilled water, before sealing the nozzle of said outer cylindrical therewith.

In all above-mentioned assembly orders and wetting steps the gasket and the cap are dried in clean air immediately after wetting the surface of the gasket and the cap and immediately before sealing the nozzle and the opening of the cylindrical body by means of the cap and the gasket, respectively.

### BRIEF DESCRIPTION OF THE DRAWINGS

Fig. 1 is a diagrammatical representation in section of an exemplary prefilled syringe,
Fig. 2 is a flowchart showing the process of sterilizing a prefilled syringe according to sterilization method 1 embodying the present invention, and
Fig. 3 is a flowchart indicating the process of sterilizing a prefilled syringe according to sterilization method 2 embodying the present invention.

### BEST MODE FOR CARRYING OUT THE INVENTION

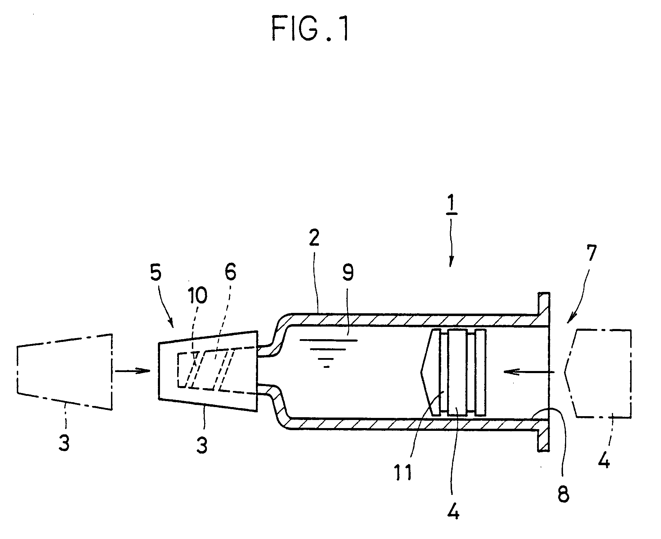

A preferred embodiment according to the invention will now be described in detail in the following, with reference to the accompanying drawings. Fig. 1 is a schematic illustration in section of an exemplary prefilled syringe 1 according to the present invention, which comprises an outer cylindrical body 2, a cap 3, and a gasket 4. The outer cylindrical body 2 includes a nozzle 6 which is integrally formed to extend from one end 5 (left side of the example as shown) of the outer cylindrical body 2 while the other end (right side of the example as shown) of the outer cylinder body 2 is kept open as an opening 8. The outer cylindrical body 2 is filled with an injection 9, which in turn is liquid-tightly sealed therein with the help of the cap 3 sealing the nozzle 6 and the gasket 4 closing the opening 8, thereby fabrication of the prefilled syringe 1 being completed.

The outer cylindrical body 2 can be made of glass or a plastic material. The outer cylindrical body 2 may be prepared in a complex structure, for instance by inserting a glass cylinder as an internal cylinder in a plastic cylinder as an exterior one.

The nozzle 6 extending from one end 5 of the outer cylindrical body 2 is formed in such a suitable shape that an injection needle (not shown) can snugly fit thereon. For securing this snug fitting, it may be possible to form a thread 10 on the surface of the nozzle 6.

The cap 3 and the gasket 4 may be made of a resilient material such as rubber, a resilient plastic and so forth. The circumferential surface of the gasket 4 may be provided with circular grooves 11 in order to enhance liquid-tightness against the injection 9 to be introduced into the outer cylindrical body 2.

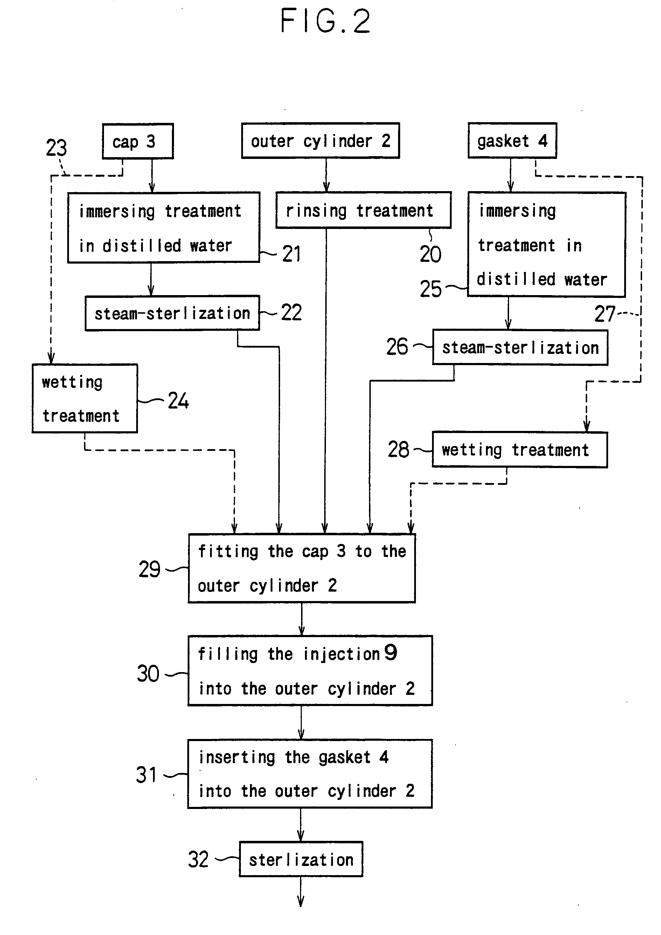

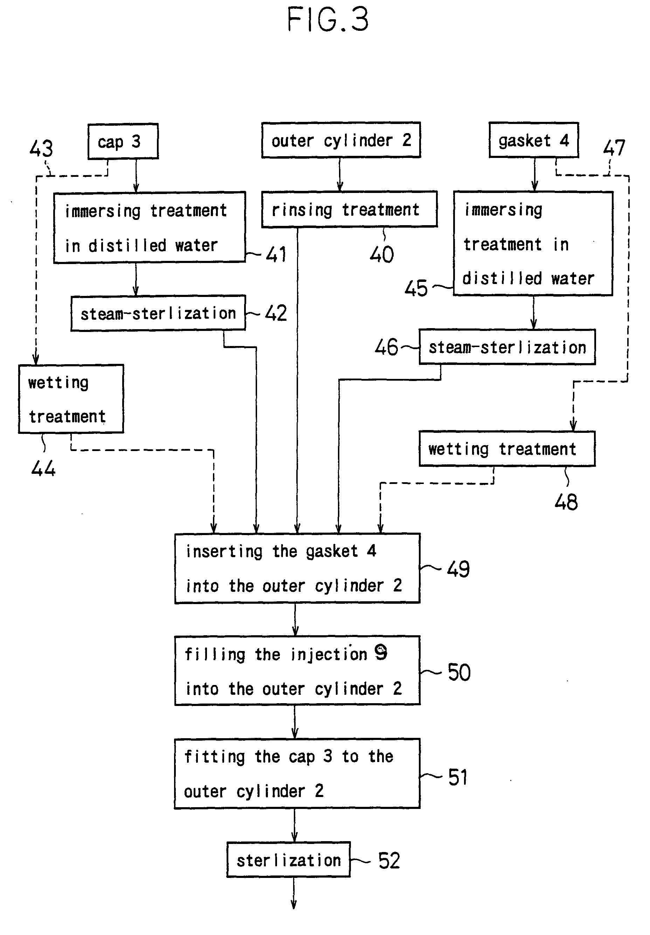

In the next, it will be described how the prefilled syringe 1 as prepared above is sterilized by means of a method of sterilization according to the present invention, with reference to flowcharts as shown in Figs. 2 and 3.

According to the flowchart as shown in Fig. 2, the outer cylindrical body 2 receives a rinsing treatment in the step 20, for instance rinsing treatment by using a high speed water jet. With this rinsing treatment, there are removed various contaminants including pyrogen which might be supposedly deposited on the surface of the outer cylinder body 2.

As to the cap 3, it is preferable to first immerse it in distilled water in the step 21 and then to sterilize it with steam in the step 22. A Koch sterilizer and an autoclave may be successfully used for steam sterilization of the cap 3.

If it is allowed the above sterilizing steps to be simplified or to take a shortcut in part, the surface of the cap 3 may be wetted in the step 24, as indicated by a dotted line 23 in Fig. 2, by either way of immersing the cap in distilled water, or exposing the cap to steam, or applying the steam sterilization to the cap as is being kept in distilled water. If it is desired to sterilize the cap 3 with steam in the step 24, the Koch sterilizer and the autoclave would suitably and successfully work for that purpose.

As to the gasket 4, it is preferable to first immerse it in distilled water in the step 25 and then to sterilize it with steam in the step 26. The Koch sterilizer and an autoclave may also be successfully used for steam sterilization of the gasket 4.

If it is allowed the above sterilization steps to be simplified or to take a shortcut in part, the surface of the gasket 4 may be wetted in the step 28, as indicated by a dotted line 27 in Fig. 2, by either way of immersing the gasket in distilled water, or exposing the gasket to steam, or applying the steam sterilization to the gasket as is being kept in distilled water. If it is desired to sterilize the gasket 4 with steam in the step 28, the Koch sterilizer and the autoclave would suitably and successfully work for that purpose.

In the step 29, the cap 3 which has been immersed in distilled water in the step 21 and sterilized with steam in the step 22 or made to wet in the step 24, is fitted to the nozzle 6 of the outer cylindrical body 2 which has been rinsed in the step 20. It may be also possible to take the following steps before fitting said cap 3 to the nozzle 6, namely the steps of placing the cap 3 on a clean bench (not shown) to dry it in a clean air shower after applying steam sterilization thereto in the step 22 or wetting it in the step 24, and then fitting it to the nozzle 6 in the step 29. In this way, the nozzle 6 as formed at one end 5 of the outer cylinder body 2 is sealed with the cap 3 in the step 29. In the next step 30, the inside of the outer cylindrical body 2 is filled with the injection 9 as introduced through the opening 8 provided at the other end 7 of the outer cylindrical body 2.

In the next step 31, the gasket 4 is inserted, usually under the circumstance at a reduced pressure, in the outer cylindrical body 2 to liquid-tightly close its opening 8. Before this gasket insertion into the outer cylindrical body 2, the gasket 4 has been immersed in distilled water in the step 25 and has received steam sterilization in the step 26 or has been wetted in the step 28. It may be also possible to take the following steps before inserting the gasket in the opening 8 of the outer cylindrical body 2, namely the steps of placing the gasket 4 on a clean bench (not shown) to dry it in a clean air shower after sterilizing it in the step 26 or wetting it in the step 28, and then, inserting it in the opening 8 of the outer cylindrical body 2 in the step 31.

In this way. fabrication of the prefilled syringe has been completed through respective steps up to the step 31. This fabricated prefilled syringe 1 is then entirely sterilized in the next step 32. In this step 32, it is also possible to perform steam sterilization over the entirety of the prefilled syringe 1 by using the Koch sterilizer or the autoclave. The condition of steam sterilization should be varied depending on the size and material of the outer cylindrical body 2 and also on the shape and material of the cap 3 and the gasket 4, but it is preferable that they are designed to stand the sterilizing process at a temperature of 121°C for about 30 min. As a matter of course, the inner pressure of the outer cylindrical body 2 is elevated with rise of the temperature of the injection 9, and this pressure increase causes the gasket 4 to be pushed out of the outer cylindrical body, eventually. In order to prevent the gasket 4 from running off the cylindrical body 2, especially in case of the plastic cylindrical body, it is necessary to suitably control the pressure in the sterilization chamber.

In the next, another sterilization method of the prefilled syringe according to the present invention will be described with reference to Fig. 3. In the same way as in the abovementioned method 1, the outer cylindrical body 2 is rinsed to remove contaminants deposited on the surface of the outer cylindrical body 2 in the step 40. It is preferable that the cap 3 receives steam sterilization in the step 42 after it is immersed in distilled water. If it is allowed to simplify or omit some of sterilizing steps in part, however. it may be possible to wet the cap 3 in the step 44, as indicated by a dotted line 43 in Fig. 3, by either way of immersing the cap in distilled water, or exposing the cap to steam, or giving steam sterilization to the cap as is being kept in distilled water. Regarding the gasket 4, it is preferable that it is first immersed in distilled water in the step 45 and is then sterilized with steam in the step 46. If it is allowed to simplify or neglect some steps of the sterilizing process in part, the gasket 4 may be wetted in the step 48, as indicated by a dotted line 47 in Fig. 3, by either way of immersing the gasket in distilled water, or exposing the gasket to steam, or sterilizing the gasket with steam as is being kept in distilled water.

In the step 49, the gasket 4 is inserted in the outer cylindrical body 2 which has been already rinsed in the step 40, through its opening 8. Before inserting the gasket in the outer cylindrical body 2, the gasket 4 has been immersed in distilled water in the step 45 and received steam sterilization in the step 46, or has been made wet in the step 48. It may be also possible to take the following steps before this gasket insertion to the opening 8 of the outer cylindrical body 2, namely the steps of placing the gasket 4 on a clean bench (not shown) to dry it in a clean air shower after applying steam sterilization to the gasket in the step 46 or wetting it in the step 48 and then, inserting it in the opening 8 of the outer cylindrical body 2 in the step 49. In the way as described above, the opening 8 of the outer cylindrical body 2 is sealed with the gasket 4 in the step 49. Then, the injection 9 is introduced in the outer cylindrical body 2 through its nozzle 6 in the next step 50.

In the next step 51, the cap 3 which has been immersed in distilled water in the step 41 and sterilized with steam in the step 42, or has been made wet in the step 44, is fitted to the nozzle 6 of the outer cylindrical body 2 which has been rinsed in the step 40. It may be also possible to take the following steps before fitting the nozzle 6 to the cap 3, namely the steps of placing the cap 3 on a clean bench (not shown) to dry it in a clean air shower after sterilizing it with steam in the step 42 or wetting it in the step 44 and then, fitting it to the nozzle 6 in the step 51.

In this way, fabrication of the prefilled syringe has been completed through respective steps up to the step 51. This fabricated prefilled syringe 1 is then entirely sterilized in the next step 52. In this step 52, it is also possible to perform steam sterilization over the entirety of the prefilled syringe 1 by using the Koch sterilizer or the autoclave. As the condition for steam sterilization, it may be possible to set the processing temperature and time for sterilization, for instance as 121°C and 30 min.

The following is the description about a comparison study which was carried out in order to prove how advantageous the method of the present invention is. The comparison study was carried out with regard to two groups of prefilled syringe test pieces. One group (referred to as Group G1 hereinafter) was prepared by applying the method of the present invention while the other (referred to as Group G2 hereinafter) was prepared without applying the same.

For preparing test pieces of Group G1, both caps and the gaskets which were made of rubber, were first immersed in distilled water for about 30 sec., and then sterilized with steam at a temperature of 121°C for about 20 min with the help of the autoclave. As to the gasket, two types of them were prepared, one being the type A gasket which makes a relatively less close contact with the inside wall of the outer cylindrical body and the other being the type B gasket which makes a relatively much more close contact with the same. These caps and baskets were left on the clean bench for about 1 hour after sterilization thereof. Then, the sterilized cap was fitted to the nozzle to close one end of the outer cylindrical body. When fitting the cap to the nozzle, a strip of biological indicator (a product of 3M, USA) was inserted in the gap between the inside surface of the cap and the outside surface of the nozzle. After fitting the cap to the nozzle, distilled water of 100 ml was introduced in the outer cylindrical body and the sterilized gasket was inserted in the outer cylindrical body at a reduced pressure to close the other end of the outer cylindrical body, thereby finalizing the fabrication of the syringe test piece according to the invention. When inserting the gasket in the outer cylindrical body, a strip of biological indicator was also inserted in the gap between the circumferential surface of the gasket and the inside surface of the outer cylindrical body. Three each of the syringe test pieces were prepared corresponding to each of gasket types A and B.

On the other hand, in the preparation of the test pieces for Group G2, caps and gaskets were neither immersed in distilled water nor sterilized with steam. In the same way as in the Group G1, two types of gaskets were prepared as types A and B. Distilled water of 100 ml was introduced in the outer cylindrical body after fitting the cap to the nozzle and then, the opening of the outer cylindrical body was sealed with the gasket at a reduced pressure, thereby finalizing the preparation of test pieces for Group G2. In the same way as in the Group G1, a strip of biological indicator is inserted in the gap between the inside surface of the cap and the outside surface of the nozzle as well as the gap between the circumferential surface of the gasket and the inside wall of the outer cylindrical body. Also, three each of test pieces were prepared corresponding to each of gasket types A and B.

These syringe test pieces of Groups G1 and G2 were sterilized with steam at a temperature of 121°C for 30 min. After performing this sterilization, each strip was taken out and placed in a culture device to cultivate *bacillus stearothermophilus* as a test bacillus under a predetermined culture condition. The cultivation results i.e. growth state of bacillus stearothermophilus are indicated in Table 1.

**Table 1**

| Test Piece | Cap | Gasket | |
|---|---|---|---|
| | | Type A | Type B |
| Group G1 | ○ | ○ | ○ |
| Group G2 | × | × | × |
| ○ : No bacillus growth observed. × : Bacillus growth observed. | | | |

As will be noted from the Table 1 above, no growth of bacilli was observed in the syringe of Group G1. This proves that complete sterilization has been carried out by the method according to the invention. Contrary to this, bacilli grew in case of the syringe of Group G2. This indicates that sterilization was performed incompletely.

In the next, the relationship between the effect and the time of sterilization was investigated with regard to the case where prefilled syringe test pieces with non-sterilized cap and gasket i.e. the test pieces of Group G2 were sterilized with steam at a temperature 121°C by using the autoclave. The results of this investigation are shown in Table 2.

**Table 2**

| Condition of Sterilization | Cap | Gasket | |
|---|---|---|---|
| | | Type A | Type B |
| 121°C 30min | × | × | × |
| ditto 60min | - | ○ | × |
| ditto 90min | ○ | ○ | × |
| ditto 120min | - | ○ | ○ |
| ○ : No bacillus growth observed. × : Bacillus growth observed. | | | |

As will be understood from this Table 2, in case of the test pieces of Group G2, in order to completely sterilize the gap between the circumferential surface of the gasket and the inside wall of the outer cylindrical body, it takes 60 min in case of using the gasket of the type A while it takes 180 min in case of adopting the gasket of the type B. In short. it takes twice to six times longer as compared with the time required to sterilize test pieces of Group G1. Similarly, in order to completely sterilize the gap between the inside surface of the cap and the outside surface of the nozzle, it takes 90 min, namely it takes 3 times longer comparing to the time necessary for sterilizing test pieces of Group G1.

### INDUSTRIAL APPLICABILITY

According to the present invention, the gap between the inside surface of the cap and the outside surface of the nozzle and the same between the circumferential surface of the gasket and the inner wall of the syringe can be adequately sterilized under the ordinary sterilizing condition which is applicable to the prefilled syringe.

## Claims

1. A method of sterilizing a prefilled syringe (1) having an outer cylindrical body (2) which is provided with a nozzle (6) which is integrally formed at its one end (5) and an opening (8) which is formed at its other end, comprising the steps of:
- wetting the surface of a cap (3) by either way of immersing (24; 44) said cap (3) in distilled water, or exposing (24; 44) the same to steam, or applying steam sterilization (24; 44) to the same while keeping the same in distilled water, or applying steam sterilization (22; 42) to the same after having immersed (21; 41) the same in distilled water,
- wetting the surface of a gasket (4) by either way of immersing (28; 48) said gasket (4) in distilled water, or exposing (28; 48) the same to steam, or applying steam sterilization (28; 48) to the same while keeping the same in distilled water, or applying steam sterilization (26; 46) to the same after having immersed (25; 45) the same in distilled water,
- sealing either the nozzle (6) or the opening (8) of said cylindrical body (2) by means of the cap (3) and the gasket (4), respectively,
- introducing a medical liquid substance (9) into the inside of said outer cylindrical body (2),
- fabricating the prefilled syringe (1) by sealing either the opening (8) or the nozzle (6) of said outer cylindrical body (2) with the gasket (4) and the cap (3), respectively,
- sterilizing the entirety of said prefilled syringe (1) as fabricated,
**characterized in that**:
- immediately after wetting the surface of the gasket (4) and the cap (3) and immediately before sealing the nozzle (6) and the opening (8) of the cylindrical body (2) by means of the cap (3) and the gasket (4), respectively, drying the gasket (4) and the cap (3) in clean air.

## Patentansprüche

1. Verfahren zum Sterilisieren einer vorgefüllten Spritze (1) mit einem äußeren zylindrischen Körper (2), der mit einer integral an seinem einen Ende (5) ausgebildeten Kanüle (6) und einer an seinem anderen Ende ausgebildeten Öffnung (8) ausgestattet ist, welches die folgenden Schritte umfaßt:
- Befeuchten der Oberfläche einer Kappe (3), indem entweder die Kappe (3) in destilliertes Wasser getaucht wird (24; 44), oder sie Dampf ausgesetzt wird (24; 44), oder eine Dampfsterilisation (24; 44) an ihr angewandt wird, während sie in destilliertem Wasser gehalten wird, oder indem eine Dampfsterilisation (22; 42) an ihr angewandt wird, nachdem sie in destilliertes Wasser getaucht worden ist,
- Befeuchten der Oberfläche einer Dichtung (4), indem der Dichtung (4) entweder in destilliertes Wasser getaucht (28; 48) wird, oder sie Dampf ausgesetzt wird (28; 48), oder eine Dampfsterilisation (28; 48) an ihr durchgeführt wird, während sie in destilliertem Wasser gehalten wird, oder indem eine Dampfsterilisation (26; 46) an ihr durchgeführt wird, nachdem sie in destilliertes Wasser getaucht worden ist (25; 45),
- entweder die Kanüle (6) oder die Öffnung (8) des zylindrischen Körpers (2) mittels der Kappe (3) bzw. der Dichtung (4) verschlossen wird,
- eine medizinische flüssige Substanz (9) ins Innere des äußeren zylindrischen Körpers (2) eingefüllt wird,
- die vorgefüllte Spritze (1) gefertigt wird, indem entweder die Öffnung (8) oder die Kanüle (6) des äußeren zylindrischen Körpers (2) mit der Dichtung (4) bzw. der Kappe (3) verschlossen wird,
- die gesamte so gefertigte vorgefüllte Spritze sterilisiert wird,
**dadurch gekennzeichnet, daß**:
- unmittelbar nach Befeuchten der Oberfläche der Dichtung (4) und der Kappe (3) und unmittelbar vor Verschließen der Kanüle (6) und der Öffnung (8) des zylindrischen Körpers (2) mit der Kappe (3) bzw. der Dichtung (4) die Dichtung (4) und die Kappe (3) in reiner Luft getrocknet werden.

## Revendications

1. Procédé de stérilisation d'une seringue préremplie (1) ayant un corps cylindrique externe (2) qui est muni d'une busette (6) qui est solidairement formée à sa première extrémité (5) et d'une ouverture (8) qui est formée à son autre extrémité, comprenant les étapes consistant à :
◆ mouiller la surface d'un bouchon (3) soit à l'aide de l'immersion (24 ; 44) dudit bouchon (3) dans de l'eau distillée, soit en exposant (24 ; 44) celui-ci à la vapeur, soit en lui appliquant une stérilisation par vapeur (24; 44) en le maintenant dans de l'eau distillée, soit en appliquant la stérilisation par vapeur (22 ; 42) à celui-ci après l'avoir immergé (21, 41) dans de l'eau distillée,
◆ mouiller la surface d'un joint (4) soit à l'aide de l'immersion (28, 48) dudit joint (4) dans de l'eau distillée, soit en l'exposant (28, 48) à la vapeur, soit en appliquant la stérilisation par vapeur (28, 48) à celui-ci tout en le maintenant dans de l'eau distillée, soit en lui appliquant la stérilisation par vapeur (26, 46) après l'avoir immergé (25 ; 45 ) dans de l'eau distillée,
◆ fermer soit la busette (6), soit l'ouverture (8) dudit corps cylindrique (2) au moyen du bouchon (3) et dudit joint (4), respectivement,
◆ introduire une substance liquide médicale (9) à l'intérieur dudit corps cylindrique externe (2),
◆ fabriquer la seringue préremplie (1) en fermant soit l'ouverture (8), soit la busette (6) dudit corps cylindrique externe (2) avec le joint (4) et le bouchon (3), respectivement,
◆ stériliser la totalité de ladite seringue préremplie (1) telle que fabriquée,
**caractérisé en ce qu'**immédiatement après avoir mouillé la surface du joint (4) et du bouchon (3) et immédiatement avant de fermer la busette (6) et l'ouverture (8) du corps cylindrique (2) au moyen du bouchon (3) et du joint (4), sécher respectivement le joint (4) et le bouchon (3) dans de l'air stérile.
